# EUROPEAN PATENT APPLICATION

(11) **EP 2 815 731 A1**
(43) Date of publication of application: **24.12.2014**
(21) Application number: 13172470.0
(22) Date of filing: 18.06.2013
(51) Int. Cl.: A61F 13/00, A61F 13/02

(54) **Fluid transport dressing**

(71) Applicant: Mölnlycke Health Care AB, 402 52 Göteborg (SE)
(72) Inventor: Melin, Daniel, 413 08 Göteborg (SE); Halldin, Kristina, 444 97 Svenshögen (SE)
(74) Representative: Valea AB

(57) **Abstract**

The present disclosure relates to a fluid transport dressing (16) for a negative pressure wound treatment system (10). The fluid transport dressing (16) comprises a plurality of sealed chambers (28) comprising chamber fluid.

## Description

### TECHNICAL FIELD

The present disclosure relates to a fluid transport dressing. Furthermore, the present disclosure relates to a negative pressure abdominal wound treatment system kit. Moreover, the present disclosure relates to a bridging kit for a negative pressure wound treatment system. Further, the present disclosure relates to a negative pressure wound treatment system. Additionally, the present disclosure relates to a use of a fluid transport dressing.

### BACKGROUND

Some types of wounds are advantageously treated by so called negative pressure wound therapy. In the field of negative pressure wound therapy, a negative pressure is applied to the wound for a relatively long time and it has been realized that the healing process may be expedited by using such a wound therapy. Moreover, for instance when treating abdominal wounds, it may be desired to remove liquid from the abdomen by using a negative pressure wound therapy system.

A negative pressure wound therapy system may be used which may comprise a fluid transport dressing. The fluid transport dressing may in some applications be adapted to be placed on or by an area of the wound to be treated by the system. In other applications, the fluid transport dressing may be adapted so as to provide a fluid transport from the wound area to a fluid port that is adapted to be connected to a negative pressure source. A system using the latter application may be referred to as a bridging system.

US2012/0136326 A1 proposes that, within the field of abdominal wounds, a dressing be used that includes two films with fluid transport conduits located therebetween. However, the design of the US2012/0136326 A1 dressing is relatively complex with various components which may result in a relatively expensive dressing that may also be cumbersome to handle.

### SUMMARY

One object of the present disclosure is to provide a fluid transport dressing for a negative pressure wound treatment system, which fluid transport dressing is cost efficient to manufacture but which nevertheless provides an appropriate fluid transport therethrough.

This object is achieved by a fluid transport dressing according to claim 1.

As such, the present disclosure relates to a fluid transport dressing for a negative pressure wound treatment system. The fluid transport dressing comprises a plurality of sealed chambers comprising chamber fluid.

A fluid transport dressing according to the above thus comprises sealed cavities comprising chamber fluid. Such sealed cavities will generally not be compressed when subjected to a negative pressure and this implies that the fluid transport dressing may maintain its fluid transporting capability even when being used in a negative pressure wound treatment system. Moreover, when subjected to a negative pressure, the sealed cavities may not expand to such an extent that the thus expanded sealed cavities together will hinder fluid transport through the fluid transport dressing. As such, the fluid transport dressing according to the present disclosure has a relatively low risk of becoming clogged during use.

Moreover, the sealed cavities may provide a cushioning effect to the site at which it is used, for instance at or by a wound, which consequently may provide a more pleasant treatment procedure for the person who is subjected to the negative pressure wound therapy.

Optionally, the fluid transport dressing is such that when the fluid transport dressing is subjected to a negative pressure the absolute value of which is less than or equal to 20 mmHg, at least one fluid conduit is provided between the sealed chambers.

As used herein, the abbreviation "mmHg" relates to the pressure unit millimeter of mercury. One millimeter of mercury is approximately 133.3 Pa (Pascals).

Optionally, the fluid transport dressing comprises a plurality of permanently sealed chambers comprising chamber fluid.

Optionally, the at least one fluid conduit is provided within the fluid transport dressing between the sealed chambers. Alternatively, the at least one fluid conduit may be provided between a top sheet and a bottom sheet of the fluid transport dressing.

Optionally, the fluid transport dressing comprises a sheet of plastics material.

Optionally, the plastics material comprises polyurethane.

Optionally, the chamber fluid is a gas.

Optionally, the chamber fluid has a chamber pressure that is within the range of 80 to 120 kPa. The chamber pressure within the above pressure range implies that the chambers may provide an appropriate cushioning effect. Moreover, a chamber pressure within the above range implies that the cavities may undergo only a limited expansion when subjected to a negative pressure.

Optionally, a minimum distance between two adjacent sealed chambers is at least 1 mm. The above discussed minimum distance implies that a fluid transport through the dressing is enabled even if the cavities expand when subjected to negative pressure.

Optionally, each one of the sealed chambers encloses a volume of at least 10 mm³.

Optionally, the fluid transport dressing comprises a first set of sealed chambers and a second set of sealed chambers. Each one of the sealed chambers of the first set of sealed chambers is adapted to rupture when subjected to a negative pressure, the absolute value of which is equal to or above a first predetermined threshold value. Each one of the sealed chambers of the second set of sealed chambers is adapted not to rupture when subjected to a negative pressure, the absolute value of which is equal to the first predetermined threshold value.

In a negative pressure system, there may be a risk of clogging of one or more conduits that provides a fluid communication between the fluid transport dressing and a source of negative pressure, such as a vacuum pump. By virtue of the fluid transport dressing as described hereinabove, the first set of sealed chambers may be adapted to burst within a desired negative pressure range, resulting in that chamber fluid may be transported towards the source of negative pressure via the one or more conduits. The chamber fluid thus transported via the one or more conduits may assist in preventing clogging thereof.

Optionally, each one of the sealed chambers of the second set of sealed chambers is adapted to rupture when subjected to a negative pressure, the absolute value of which is equal to or above a second predetermined threshold value. The first predetermined threshold value is different from the second predetermined threshold value.

Optionally, the fluid transport dressing is sterilized.

Optionally, the negative pressure wound treatment system is adapted for use with abdominal wounds.

Optionally, the dressing comprises a top sheet and a bottom sheet and a plurality of sealed chambers located therebetween, the top sheet comprising a top sheet portion that is liquid permeable. The bottom sheet comprises a first bottom sheet portion that is liquid impermeable and a second bottom sheet portion that is liquid permeable. The second bottom sheet portion at least partially surrounds the first bottom sheet portion.

With a fluid transport dressing according to the above, liquid may enter the dressing via the liquid permeable top sheet or the liquid permeable second bottom sheet portion. Moreover, the first bottom sheet portion, which is liquid impermeable, may be adapted to be placed directly upstream of a negative pressure wound treatment component that is adapted to contribute to a fluid communication between the fluid transport dressing and a source of negative pressure. Purely by way of example, such a component may be a foam.

Generally, the absolute value of the negative pressure directly upstream of a negative pressure wound treatment component is higher than the absolute value of the negative pressure of a portion of the fluid transport dressing that is located at a distance from the negative pressure wound treatment component. This may in turn increase the risk of having e.g. tissue from the wound that is to be treated sucked into the fluid transport dressing. Such a risk is reduced by virtue of the above dressing since the first bottom sheet portion is liquid impermeable.

A second aspect of the present disclosure relates to a negative pressure abdominal wound treatment system kit. The kit comprises a fluid transport dressing according to the first aspect of the present disclosure. The kit further comprises a fluid transport member, for instance a foam and/or a gauze, and a wound cover member. The fluid transport dressing is adapted to be applied to a wound floor of the abdominal wound.

Optionally, the fluid transport member is adapted to be located in a space between the wound floor and an abdominal wall.

Optionally, the wound cover member foam is adapted to cover the abdominal wound.

A third aspect of the present disclosure relates to a bridging kit for a negative pressure wound treatment system. The bridging kit comprises a fluid transport assembly adapted to provide a fluid transport between a first fluid port, adapted to be connected to a negative pressure source, and a second fluid port, adapted to be connected to a wound cover member adapted to cover the wound. The fluid transport assembly comprises the fluid transport dressing according to the first aspect of the present disclosure.

A bridging kit as presented hereinabove, may be used for providing a distance between the area of the wound to be treated and a first fluid port that is adapted to be connected to a negative pressure source. The first fluid port may be connected to the negative pressure source by means of a fluid communication assembly that may comprise fluid communication components such as a conduit and/or a suction device. Depending on the location and/or the type of wound to be treated, such fluid communication components may cause discomfort to the recipient of the negative pressure wound therapy. Thus, it may be advantageous if the fluid communication components be located at a distance from the wound area. Moreover, the wound to be treated by the negative pressure wound therapy may be located in a position that is difficult to access and, in such a scenario, it may also be beneficial to use a bridging kit.

The bridging kit as discussed hereinabove, which comprises a fluid transport dressing according to the first aspect of the present disclosure, implies that a bridging system may be obtained, which system has a low risk of becoming clogged during use. Moreover, since the bridging system may be used for treating wounds that are sensitive to contact forces, the above discussed cushioning effect of the fluid transport dressing may contribute to a more comfortable therapy.

Optionally, the fluid transport assembly further comprises a fluid transport cover that is adapted to cover the fluid transport dressing.

A fourth aspect of the present disclosure relates to a negative pressure wound treatment system. The system comprises a fluid transport dressing according to the first aspect of the present disclosure and/or a negative pressure abdominal wound treatment system kit according to the second aspect of the present disclosure and/or a bridging kit according to the third aspect of the present disclosure.

A fifth aspect of the present disclosure relates to negative pressure wound treatment system. The fluid transport dressing comprises a plurality of sealed chambers comprising chamber fluid.

### BRIEF DESCRIPTION OF THE DRAWINGS

With reference to the appended drawings, below follows a more detailed description of embodiments of the invention cited as examples.

In the drawings:
- Fig. 1: illustrates an embodiment of a negative pressure wound therapy system;
- Fig. 2: illustrates a side view of an embodiment of a portion of a fluid transport dressing;
- Fig. 3: illustrates a top view of the Fig. 2 fluid transport dressing;
- Fig. 4: illustrates another embodiment of the fluid transport dressing;
- Fig. 5: illustrates an exploded view of a further embodiment of the fluid transport dressing;
- Fig. 6: illustrates a further embodiment of the fluid transport dressing, and
- Fig. 7: illustrates a negative pressure wound treatment system that comprises a bridging kit.

It should be noted that the appended drawings are not necessarily drawn to scale and that the dimensions of some features of the present invention may have been exaggerated for the sake of clarity.

### DETAILED DESCRIPTION OF PREFERRED EMBODIMENTS

The invention will, in the following, be exemplified by embodiments. It is to be understood, however, that the embodiments are included in order to explain principles of the invention and not to limit the scope of the invention defined by the appended claims.

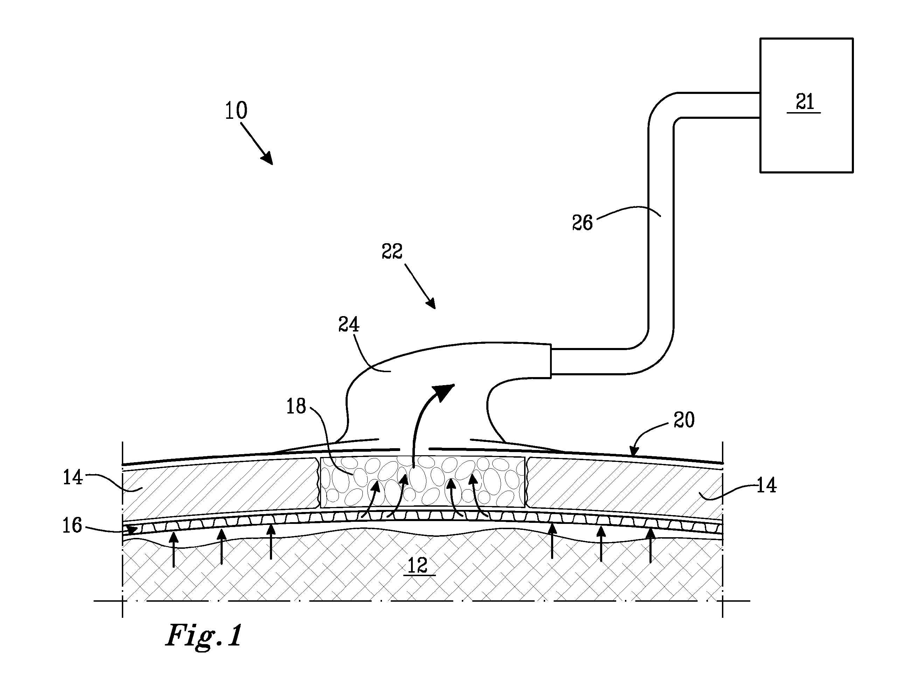

Fig. 1 illustrates a negative pressure wound therapy system 10. In Fig. 1, the system 10 is exemplified as a negative pressure abdominal wound treatment system.

As such, the Fig. 1 system 10 is adapted for use with abdominal wounds. Fig. 1 illustrates a wound floor 12 as well as an abdominal wall 14 of the abdominal wound.

The negative pressure wound therapy system 10 illustrated in Fig. 1 comprises a fluid transport dressing 16 at least a portion of which is adapted to be located between the wound floor 12 and the abdominal wall 14. Furthermore, the Fig. 1 system 10 comprises a fluid transport member 18 adapted to provide a fluid transport between the fluid transport dressing 16 and a negative pressure source 21.

Purely by way of example, the fluid transport member 18 may comprise a foam material, for instance an open-celled foam material. As a non-limiting example, the fluid transport member 18 may comprise a flexible open-celled foam material, such as a sponge material. One advantage of using a fluid transport member 18 that comprises a foam material may be that the foam material enables an appropriate granulation of the edges of the abdominal wall 14. Examples of suitable foam materials include, without limitation, materials comprising polyurethane, polyester, polyether or polyvinyl alcohol or combinations thereof. As a non-limiting example, the foam may be a hydrophobic polymer foam.

As another non-limiting example, the fluid transport member 18 may comprise gauze.

Moreover, although purely by way of example, the negative pressure source 21 may comprise a negative pressure pump which may be referred to as a vacuum pump. Purely by way of example, the negative pressure source 21 may be adapted to provide a negative pressure, the absolute value of which is greater than or equal to a threshold value. Generally, the threshold value in such embodiments is at least 20 mmHg.

In some embodiments, the negative pressure source 21 is adapted to provide negative pressure at one fixed threshold value.

In some embodiments, the negative pressure source 21 is adapted to provide negative pressure at multiple values which may be selected, for example, by the user and/or depending on the therapy mode. In some such embodiments, the negative pressure source 21 is adapted to provide negative pressure at various values within a range. In some such embodiments, the negative pressure source 21 is adapted to provide negative pressure at any value in certain increments from a lower limit (absolute value) to an upper limit (absolute value).

In some embodiments, the negative pressure source 21 is adapted to provide negative pressure continuously during treatment. In some embodiments, the negative pressure source 21 is adapted to provide negative pressure intermittently during treatment. In some embodiments, the negative pressure source 21 is adapted to provide negative pressure either continuously or intermittently during treatment, as selected by the user.

Generally, the negative pressure source 21 is adapted to provide negative pressure at one or more values that fall within the range between about 20 mmHg and about 400 mHg (inclusive of endpoints).

For example, typical threshold values used during negative pressure wound therapy include any value in the range between about 20 mmHg and about 400 mmHg (inclusive of both endpoints), for example, about 20 mmHg, about 25 mmHg, about 50 mmHg, about 60 mmHg, about 80 mmHg, about 120 mmHg, about 200 mmHg, or about 300 mmHg. For example, in some embodiments, a negative pressure of about 80 mm Hg is used. In some embodiments, a negative pressure of about 120 mmHg is used.

The selection of the appropriate values may be made, for example, by a clinician. The choice of appropriate negative pressure value(s) may be influenced by any or a combination of factors such as location of wound, type of wound, wound healing status, type and/or material of wound filler, type of dressing, patient, etc. For example, in some embodiments where gauze is used as a wound filler, a negative pressure of about 80 mmHg is used. As a further example, in some embodiments where a foam is used as a wound filler, a negative pressure of about 120 mmHg is used.

The Fig. 1 system 10 further comprises a wound cover member 20. The wound cover member is generally adapted to be attached to the skin surrounding the wound. Purely by way of example, the wound cover member 20 may comprise a wound cover film. The wound cover member 20 may preferably be attached to the skin surrounding the abdominal wound, for instance by means of an adhesive. Examples of adhesives that may be used include, but are not limited to, acrylic adhesives and/or silicone gel adhesives. In some embodiments, the adhesive or adhesives is/are already incorporated as part of the wound cover film. In some embodiments, the adhesive or adhesives is/are applied to the wound cover member during use. Purely by way of example, the adhesive sold under the trademark Mepiseal® by Mölnlycke Healthcare AB may be used for attaching the wound cover member to the skin surrounding the wound.

Fig. 1 further illustrates that the negative pressure wound therapy system 10 comprises a fluid communication assembly 22 adapted to provide a fluid communication between the negative pressure source 21 and the wound cover member 20.

The fluid communication assembly 22 may for instance comprise a suction device 24 and a conduit assembly 26 comprising one or more conduits.

Arrows in Fig. 1 indicate how liquid may travel from the abdominal wound towards the negative pressure source 21 via the fluid transport dressing 16, the fluid transport member 18 and the fluid communication assembly 22, respectively.

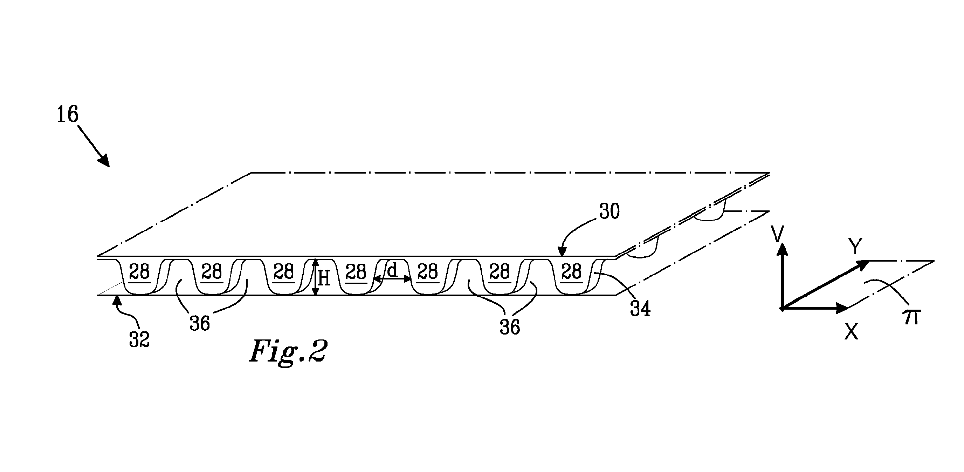

Fig. 2 illustrates an embodiment of a fluid transport dressing 16 according to the present invention. As may be gleaned from Fig. 2, the fluid transport dressing 16 comprises a plurality of sealed chambers 28 comprising chamber fluid.

The fluid transport dressing 16 may have a planar extension in a first plane π and the fluid transport dressing may also extend in a vertical direction V that is perpendicular to the extension of the first plane π.

The Fig. 2 embodiment of the fluid transport dressing 16 comprises a top sheet 30 and a bottom sheet 32. At least one, alternatively both, of the top and bottom sheets 30, 32 may be of a plastics material which may comprise polyurethane.

The sealed chambers 28 are located between the top and bottom sheets 30, 32. In the Fig. 2 embodiment of the fluid transport dressing 16, the sealed chambers 28 are at least partially delimited by an intermediate sheet 34 that extends at least partially between the top and bottom sheets 30, 32. The intermediate sheet 34 may be deformed, for instance pleated, and thereafter attached to at least one of the top and bottom sheets 30, 32 such that the sealed chambers 28 are formed. The intermediate sheet 34 may be attached to both the top and bottom sheets 30, 32 such that a continuous fluid transport dressing 16 is obtained.

Purely by way of example, each one of the sealed chambers encloses a volume of at least 10 mm³. Alternatively, each one of the sealed chambers encloses a volume of at least 50 mm³. As another non-limiting option, each one of the sealed chambers encloses a volume of at least 80 mm³.

In other non-limiting examples of a sealed chamber, the sealed chamber may have a substantially cylindrical shape with a height, i.e. an extension in a direction perpendicular to the planar extension of the top sheet 30 and/or the bottom sheet 32, and a diameter in the planar extension. Purely by way of example, the height of a sealed chamber may be within the range of 0.3 - 0.7 of the diameter of the sealed chamber.

Purely by way of example, the sealed chamber may have a diameter that is one of the following: 3, 6, 9 or 25 mm. Assuming, as a non-limiting example, that the height of the sealed chamber is approximately half the diameter of the sealed chamber, the volume of the sealed chamber is approximately 10 mm³ for a sealed chamber with a diameter of 3 mm, approximately 80 mm³ for a sealed chamber with a diameter of 6 mm, approximately 280 mm³ for a sealed chamber with a diameter of 9 mm and approximately 6000 mm³ for a sealed chamber with a diameter of 25 mm.

As a non-limiting example, the intermediate sheet 34 may be of a plastics material which may comprise polyurethane.

Instead of, or in addition to polyurethane, at least one, alternatively two or more, of the top, bottom and intermediate sheets 30, 32, 34 may comprise at least one of the following materials: other types of urethanes, silicone, transparent hydrocolloid, PVC, hydrogel, copolyester, polyethylene, TPS (thermoplastic elastomers based on styrene) or TPO (thermoplastic olefins) i.e. blends of polyethylenes and polypropylenes.

Purely by way of example, each one of the top and bottom sheets 30, 32 as well as the intermediate sheet 34 may have a thickness within the range of 0.01 - 0.1 mm.

A procedure for making the fluid transport dressing 16 may be performed at substantially atmospheric pressure such that the sealed chambers 28 comprise fluid at substantially atmospheric pressure. Alternatively, the procedure may be performed in a closed area to which a chamber fluid is fed at a predetermined pressure. In such a procedure, the pressure in the sealed chambers 28 may be above or below atmospheric pressure. The fluid fed into the closed area may be a gas, such as air. As another alternative, the gas may be an inert gas.

Purely by way of example, the intermediate sheet 34 may be attached to the top sheet 30 and/or the bottom sheet 32 by means of an adhesive, such as glue, and/or by welding.

Fig. 2 further illustrates that the fluid transport dressing 16 comprises a plurality of voids 36 located between adjacent sealed chambers 28. The voids 36 form part of one or more fluid conduits that extends through at least a portion of the fluid transport dressing 16.

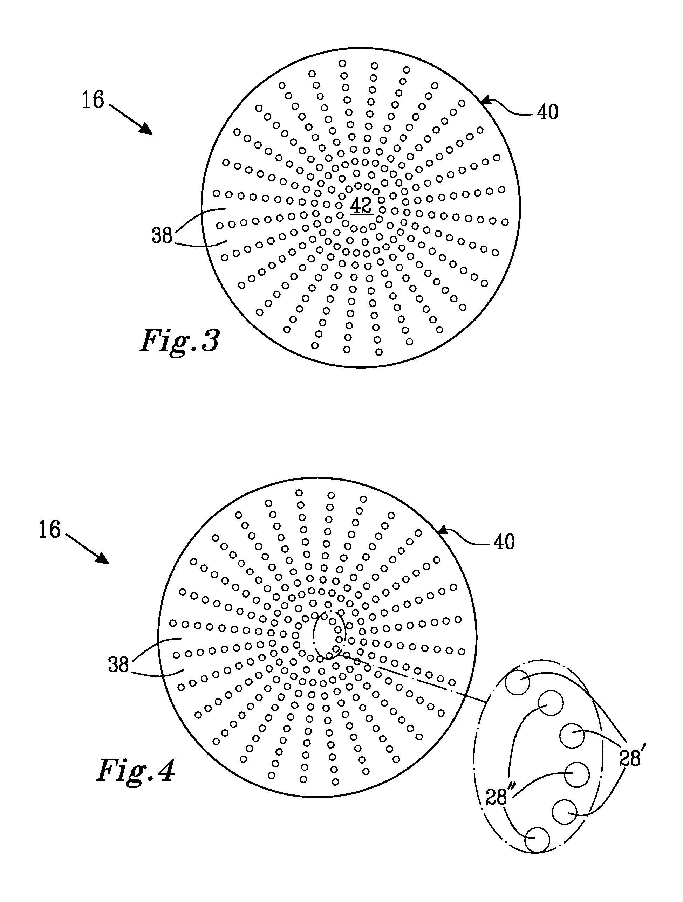

Fig. 3 illustrates a top view of the Fig. 2 fluid transport dressing 16. Fig. 3 further indicates a plurality of fluid conduits 38. In the Fig. 2 and Fig. 3 embodiment of the fluid transport dressing 16, the sealed chambers 28 are arranged such that at least a plurality of the fluid conduits 38 extend from the periphery 40 of the fluid transport dressing 16 towards the centre 42 thereof. However, in other embodiments of the fluid transport dressing 16, the sealed chambers 28 may be arranged in other ways. For example, the sealed chambers 28 may be spaced evenly throughout the fluid transport dressing. Fig. 2 and Fig. 3 further illustrate that at least one fluid conduit 38 is provided within the fluid transport dressing 16 between the sealed chambers 28.

As a non-limiting example, irrespective of the configuration of the sealed chambers 28, the fluid transport dressing 16 may be such that when the fluid transport dressing is subjected to a negative pressure, the absolute value of which is less than or equal to a fluid conduit threshold value, for instance 20 mmHg, at least one fluid conduit is provided between the sealed chambers 28.

Purely by way of example, typical fluid conduit threshold values for which at least one fluid conduit is provided between the sealed chambers 28 may include any value in the range between about 20 mmHg and about 400 mmHg (inclusive of both endpoints), for example, about 20 mmHg, about 25 mmHg, about 50 mmHg, about 60 mmHg, about 80 mmHg, about 120 mmHg, about 200 mmHg, or about 300 mmHg.

The above feature of the fluid transport dressing 16 may be obtained in a plurality of ways. Purely by way of example, the fluid transport dressing may be designed such that the chamber fluid has a chamber pressure that is within the range of 80 to 120 kPa (kiloPascals) such that the cavities undergo only a limited expansion when subjected to a negative pressure the absolute value of which is less than or equal to 20 mmHg.

Instead of, or in addition to, the above discussed pressure range for the sealed chambers 28, the fluid transport dressing 16 may be arranged such that a minimum distance d between two adjacent sealed chambers 28 is at least 1 mm, alternatively at least 1.5 mm or at least 2 mm. Any one of the above discussed minimum distances implies that a fluid transport through the dressing is enabled even if the cavities expand when subjected to negative pressure. The minimum distance d is measured at the location in the vertical direction V that is located halfway between the top sheet 30 and the bottom sheet 32. Fig. 4 illustrates an embodiment of the fluid transport dressing 16 which comprises a first set of sealed chambers 28' and a second set of sealed chambers 28". Each one the first set of sealed chambers 28' is adapted to rupture when subjected to a negative pressure, the absolute value of which is equal to or above a first predetermined threshold value P₁. Each one the second set of sealed chambers 28" is adapted not to rupture when subjected to a negative pressure, the absolute value of which is equal to the first predetermined threshold value P₁.

Purely by way of example, the first predetermined threshold value P₁ may be within the range of 20 - 40 mmHg. Fig. 4 illustrates an embodiment of the fluid transport dressing 16 wherein two sealed chambers of the first set of sealed chambers 28' are separated by at least one sealed chamber of the second set of sealed chambers 28".

Purely by way of example, typical threshold values for the first predetermined threshold value P₁ may include any value in the range between about 20 mmHg and about 400 mmHg (inclusive of both endpoints), for example, about 20 mmHg, about 25 mmHg, about 50 mmHg, about 60 mmHg, about 80 mmHg, about 120 mmHg, about 200 mmHg, or about 300 mmHg.

Purely by way of example, the first set of sealed chambers 28' is adapted to rupture when subjected to a negative pressure, the absolute value of which is equal to or above a first predetermined threshold value P₁, by weakening a portion of the film that constitutes the cavity wall for each one of the first set of sealed chambers 28'. In embodiments wherein the fluid transport dressing 16 comprises an intermediate sheet 34, portions of the intermediate sheet 34 that are adapted to form at least a portion of the cavity wall for each one of the first set of sealed chambers 28' may be weakened.

Instead of, or in addition to, weakening the cavity walls, the first set of sealed chambers 28' may be filled with chamber fluid at a first chamber pressure that exceeds a second chamber pressure of the chamber fluid of the second set of sealed chambers 28". As such, when the fluid transport dressing 16 is subjected to a negative pressure, the pressure difference will be greater for the first set of sealed chambers 28' than for the second set of sealed chambers 28" and this may in turn result in that the first set of sealed chambers 28' is more prone to bursting.

When a sealed chamber of the first set of sealed chambers 28' bursts, the chamber fluid accommodated therein will be transported towards the source of negative pressure 20 via the conduit assembly 26 for instance. A purpose of introducing the chamber fluid can be, for example, to monitor and/or dissolve a blockage or obstruction that could possibly occur in the conduit assembly 26.

Purely by way of example, the fluid of at least one sealed chamber of the first set of sealed chambers 28' may be selected in order to make it possible for a user to identify that the sealed chamber has bursted. Purely by way of example, at least one sealed chamber of the first set of sealed chambers 28' may contain a coloured fluid, such as a coloured gas, which may be identified when it travels in the conduit assembly 26. Instead of, or in addition, to using a coloured fluid, at least one of the sealed chambers of the first set of sealed chambers 28' may comprise an odorant.

In the Fig. 4 embodiment of the fluid transport dressing 16, each one of the sealed chambers of the second set of sealed chambers 28" is adapted to rupture when subjected to a negative pressure the absolute value of which is equal to or above a second predetermined threshold value P₂. The first predetermined threshold value P₁ is different from the second predetermined threshold value P₂.

The fluid transport dressing 16 according to any one of the embodiments discussed hereinabove may be sterilized before use. Purely by way of example, the fluid transport dressing 16 may be sterilized using heat, chemicals and/or radiation.

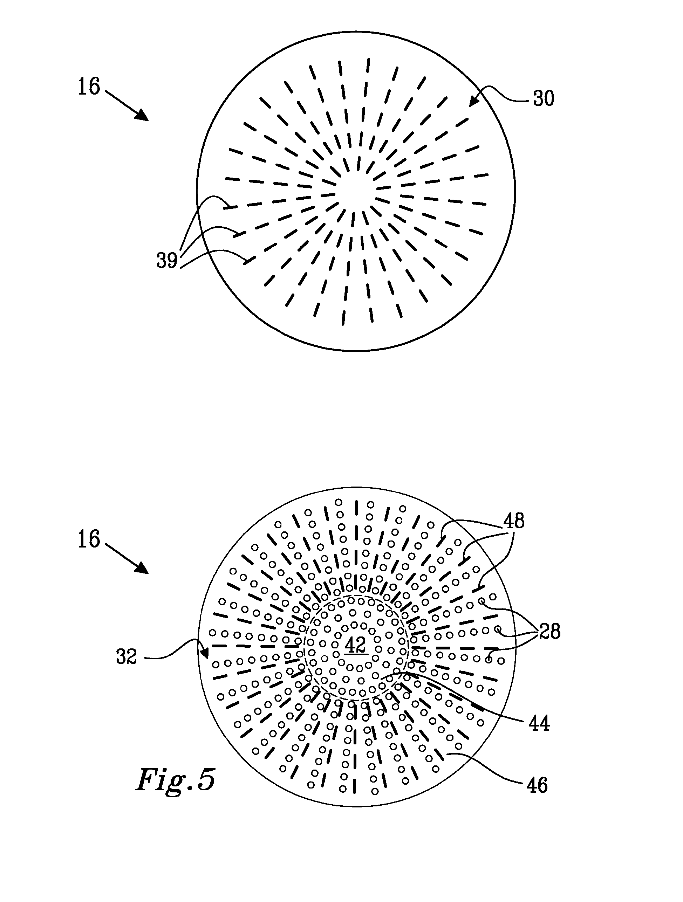

Fig. 5 illustrates an exploded view of another embodiment of a fluid transport dressing 16. The Fig. 5 embodiment of the dressing is adapted for use with abdominal wounds.

The Fig. 5 dressing comprises a top sheet 30 and a bottom sheet 32 and a plurality of sealed chambers 28 located therebetween. In the embodiment illustrated in Fig. 5, the sealed chambers 28 are at least partially formed by an intermediate sheet (not shown in Fig. 5) that is attached to the bottom sheet 32 in at least certain attachment points or attachments areas.

Moreover, in the Fig. 5 embodiment of the fluid transport dressing 16, the top sheet 30 comprises a top sheet portion that is liquid permeable. In fact, Fig. 5 illustrates an embodiment in which substantially the whole of the top sheet 30 is liquid permeable. In order to achieve the liquid permeability, the top sheet 30 may be provided with a plurality of openings such as the slits 39 illustrated in Fig. 5.

Fig. 5 further illustrates that the bottom sheet 32 comprises a first bottom sheet portion 44 that is liquid impermeable and a second bottom sheet portion 46 that is liquid permeable. The second bottom sheet portion 46 at least partially surrounds the first bottom sheet portion 44. As for the top sheet 30, the liquid permeability of the second bottom sheet portion 46 may be achieved by providing the second bottom sheet portion 46 with a plurality of openings such as the slits 48 illustrated in the Fig. 5 embodiment.

The position and size of the first bottom sheet portion 44 in relation to the second bottom sheet portion 46 may be selected such that, when the fluid transport dressing 16 is adapted for use with abdominal wounds, such as the scenario indicated in Fig. 1, a projection of the fluid transport member 18 onto the bottom sheet 32 is located with the first bottom sheet portion 44. By virtue of the size and position of the first bottom sheet portion 44 relative to the fluid transport member 18, the risk of having e.g. tissue from the wound that is to be treated sucked into the fluid transport dressing 16 may be reduced as compared to a fluid transport dressing 16 in which the whole of the bottom sheet 32 is liquid permeable.

Purely by way of example, the surface area of the first bottom sheet portion 44 may be less than 50%, alternatively less than 30%, optionally less than 20%, or less than 10%, of the surface area of the second bottom sheet portion 46. As non-limiting examples, the first bottom sheet portion 44 may have a surface area that is within the range of 20 to 300 cm², alternatively within the range of 50 to 150 cm².

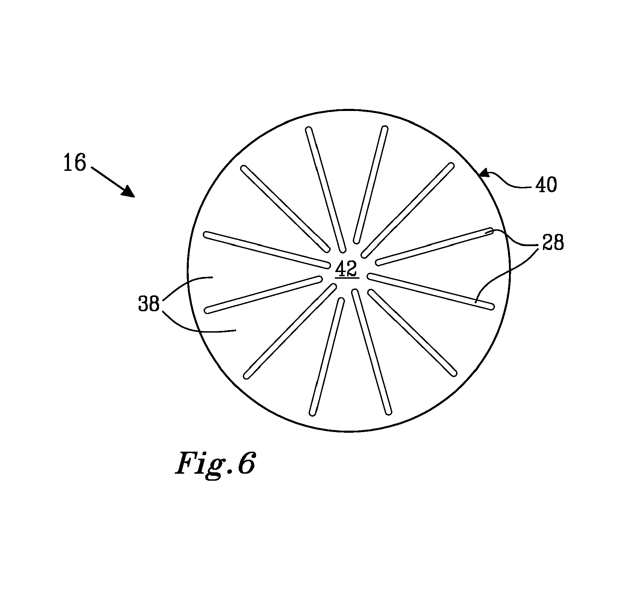

In the embodiments of the fluid transport dressing 16 illustrated in each one of Figs. 2 to 5, each one of the sealed chambers 28 has a relatively compact shape in the first plane π, i.e. the length and the width are substantially the same. However, Fig. 6 illustrates another embodiment of the fluid transport dressing 16 in which at least a plurality of the sealed chambers 28 are elongate and extend in a radial direction from the periphery 40 of the fluid transport dressing 16 towards the centre 42 thereof.

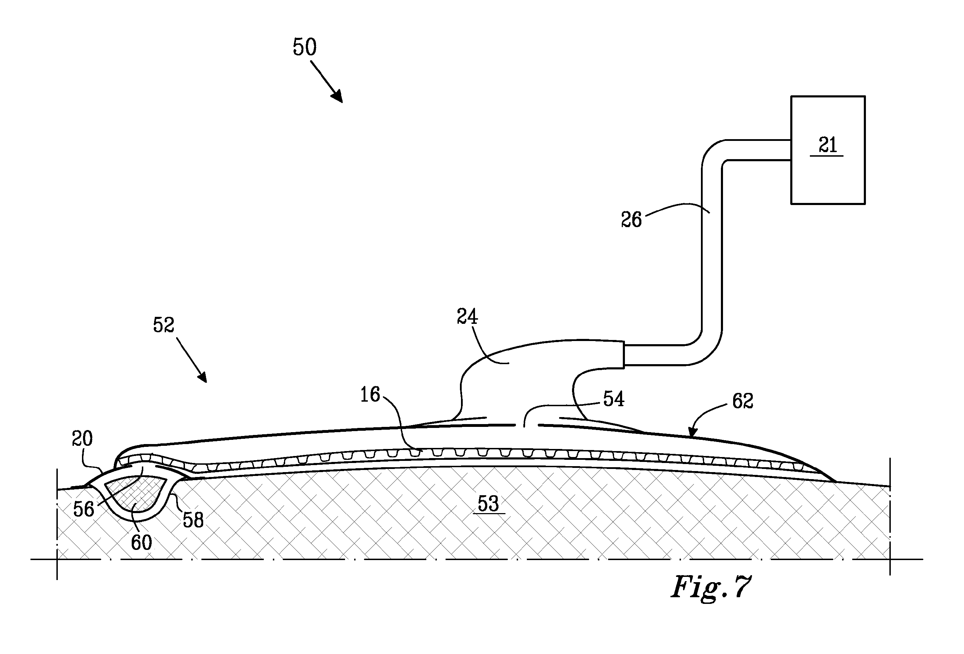

Fig. 7 illustrates another application for the fluid transport dressing 16. To this end, Fig. 7 illustrates a bridging kit 50 for a negative pressure wound treatment system 10. The bridging kit 50 comprises a fluid transport assembly 52 adapted to provide a fluid transport between a first fluid port 54, adapted to be connected to a negative pressure source 21, and a second fluid port 56, adapted to be connected to a wound cover member 20 adapted to cover a wound 58.

Fig. 7 further illustrates that the negative pressure wound treatment system 10 may comprise a wound filler 60 which is adapted to be placed on or in the wound 58 to be treated by the negative pressure wound therapy. Purely by way of example, the wound filler 60 may comprise an absorbent material, such as open-celled foam material. As a non-limiting example, the wound filler 60 may comprise a flexible open-celled foam material, such as a sponge material. In the Fig. 7 embodiment of the negative pressure wound treatment system 10, the wound cover member 20 is adapted to cover the wound filler 60.

In Fig. 7, the fluid transport assembly 52 comprises a fluid transport cover 62 that is adapted to cover the fluid transport dressing 16 as well as at least a portion of the wound cover member 20. Moreover, in the Fig. 7 embodiment, the fluid transport cover 62 comprises the first fluid port 54 whereas the wound cover member 20 comprises the second fluid port 56.

Finally, it should be recognized that structures and/or elements and/or method steps shown and/or described in connection with any disclosed form or embodiment of the invention may be incorporated in any other disclosed or described or suggested form or embodiment as a general matter of design choice.

### TEST METHOD

In order to determine whether or not at least one fluid conduit is provided between the sealed chambers of a fluid transport dressing when subjected to a negative pressure, the test method proposed hereinbelow may be used.

The fluid transport dressing to be investigated is cut in a 4x40 cm strip, wherein the length of the strip corresponds to an intended flow direction along the fluid transport dressing, and encapsulated by one or more sealing films (purely by way of example, a sealing film of a plastics material may be used) thus creating an airtight sealing around the fluid transport dressing.

An opening having a diameter of 5 mm is provided in each longitudinal end of the sealing and the sealing is placed attached to a vertically extending plate, such as acrylic plate. The vertically extending plate has openings with an opening diameter of 5 mm, i.e. a top opening and a bottom opening, that correspond to the openings of the sealing.

For each one of the openings of the sealing, a small piece of polyurethane foam is placed between the plate and the sealing in order to ensure fluid passage between the plate and the sealing. Purely by way of example, the foam sold under the trademark Avance® by Mölnlycke Healthcare AB may be used.

A top opening of the plate is connected to the negative pressure pump and the bottom opening is introduced in a body of water such that the bottom opening is within the range of 5 to 10 mm below the surface of the body of water. The pump is operated in order to produce the negative pressure of interest, for instance a negative pressure, the absolute value of which is 20 mmHg. If water continuously flows through the dressing during the application of the negative pressure, it is concluded that the fluid transport dressing is such that at least one fluid conduit is provided between the sealed chambers of a fluid transport dressing when subjected to that negative pressure.

### EXAMPLE 1

An example of a test for determining whether or not at least one fluid conduit is provided between the sealed chambers of a fluid transport dressing is presented hereinbelow.

In the below test, the following equipment was used: a negative pressure pump adapted to produce a negative pressure of 120mmHg, a bowl containing 400 mL water and an acrylic plate with holes for connecting tubes.

The fluid transport dressing to be tested had a plurality of substantially cylindrical sealed chambers with a diameter of approximately 10 mm and a height of approximately 3 mm. The spacing between two adjacent chambers was approximately 1.3 mm. The fluid transport dressing had an even distribution of sealed chambers with approximately 90 bubbles per 10 cm² area of the fluid transport dressing. The film thickness between adjacent chambers was approximately 0.05 mm and the thickness of the sealed chamber wall was approximately 0.02 to 0.03 mm.

The test was performed by firstly cutting the fluid transport dressing to be tested in a 4x40 cm strip and encapsulate in between two sheets of sealing film (in Example 1, a sealing file sold under the trademark Avance Film with Safetac technology, by Mölnlycke Healthcare AB, was used), creating an airtight sealing around the material. Two holes were cut in this sealing; one for each corresponding hole in a vertically standing acrylic plate. A small piece of Avance® foam was placed between the acrylic plate and the material tested, one for each hole to ensure fluid passage and minimize risk of blockage. The top hole on the acrylic plate was connected to the pump while the lower hole was connected to a bowl with 400mL water. A large sheet of sealing film covered the whole plate. When the pump was turned on water started to flow through the system. The time was noted every 50 mL. A total evacuation of the water in the bowl was detected after approximately 5 minutes.

### EXAMPLE 2

In order to investigate the characteristics of sealed chambers during exposure to negative pressure, a sheet of 10x10 cm fluid transport dressing was put in a sealed vacuum chamber. The fluid transport dressing tested was of the same type as the fluid transport dressing that was tested in Example 1 hereinabove. The pressure was set to -400 mmHg and the duration was 2 minutes. Once the pressure dropped, the sealed chambers expanded to a spherical shape without bursting. When the pressure returned to atmospheric level, the sealed chambers relaxed to the same shape as prior the test.

## Claims

1. A fluid transport dressing (16) for a negative pressure wound treatment system (10), said fluid transport dressing (16) comprising a plurality of sealed chambers (28) comprising chamber fluid.

2. The fluid transport dressing (16) according to claim 1, wherein said fluid transport dressing (16) is such that when said fluid transport dressing (16) is subjected to a negative pressure the absolute value of which is less than or equal to 20 mmHg, at least one fluid conduit (38) is provided between said sealed chambers (28).

3. The fluid transport dressing (16) according to claim 1 or 2, wherein said fluid transport dressing (16) comprises a sheet of plastics material.

4. The fluid transport dressing (16) according to claim 3, wherein said plastics material comprises polyurethane.

5. The fluid transport dressing (16) according to any one of the preceding claims, wherein said chamber fluid is a gas.

6. The fluid transport dressing (16) according to any one of the preceding claims, said chamber fluid has a chamber pressure that is within the range of 80 to 120 kPa.

7. The fluid transport dressing (16) according to any one of the preceding claims, wherein a minimum distance between two adjacent sealed chambers (28) is at least 1 mm.

8. The fluid transport dressing (16) according to any one of the preceding claims, wherein each one of said sealed chambers (28) encloses a volume of at least 10 mm³.

9. The fluid transport dressing (16) according to any one of the preceding claims, wherein said fluid transport dressing (16) comprises a first set of sealed chambers (28') and a second set of sealed chambers (28"), each sealed chamber of said first set of sealed chambers (28') being adapted to rupture when subjected to a negative pressure the absolute value of which is equal to or above a first predetermined threshold value (P₁), each sealed chamber of said second set of sealed chambers (28") being adapted not to rupture when subjected to a negative pressure the absolute value of which is equal to said first predetermined threshold value (P₁).

10. The fluid transport dressing (16) according to claim 9, wherein each sealed chamber of said second set of sealed chambers (28") is adapted to rupture when subjected to a negative pressure the absolute value of which is equal to or above a second predetermined threshold value, said first predetermined threshold value being different from said second predetermined threshold value (P₂).

11. The fluid transport dressing (16) according to any one of the preceding claims, wherein said fluid transport dressing (16) is sterilized.

12. The fluid transport dressing (16) according to any one of the preceding claims, wherein said negative pressure wound treatment system (10) is adapted for use with abdominal wounds.

13. The fluid transport dressing (16) according to any one of the preceding claims, wherein said fluid transport dressing (16) comprises a top sheet (30) and a bottom sheet (32) and a plurality of sealed chambers (28) located therebetween, said top sheet (30) comprising a top sheet portion that is liquid permeable, said bottom sheet (32) comprising a first bottom sheet portion (44) that is liquid impermeable and a second bottom sheet portion (46) that is liquid permeable, said second bottom sheet portion (46) at least partially surrounding said first bottom sheet portion (44).

14. A negative pressure abdominal wound treatment system kit, said kit comprising a fluid transport dressing (16) according to any one of the preceding claims, said kit further comprising a fluid transport member (18) and a wound cover member (20), said fluid transport dressing (16) being adapted to be applied to a wound floor (12) of said abdominal wound.

15. The kit according to claim 14, wherein said fluid transport member (18) is adapted to be located in a space between said wound floor and an abdominal wall.

16. A bridging kit (50) for a negative pressure wound treatment system (10), said kit comprising a fluid transport assembly (52) adapted to provide a fluid transport between a first fluid port (54), adapted to be connected to a negative pressure source (21), and a second fluid port (56), adapted to be connected to a wound cover member (20) adapted to cover said wound, said fluid transport assembly (52) comprising said fluid transport dressing (16) according to any one of claims 1 to 13.

17. The bridging kit (50) according to claim 16, wherein said fluid transport assembly (52) further comprises a fluid transport cover (62) that is adapted to cover said fluid transport dressing (16).

18. A negative pressure wound treatment system (10), said system comprising a fluid transport dressing (16) according to any one of claims 1 to 13 and/or a negative pressure abdominal wound treatment system kit according to any one of claims 14 to 15 and/or a bridging kit (50) according to any one of claims 16 or 17.

19. A use of a fluid transport dressing (16) in a negative pressure wound treatment system (10), said fluid transport dressing (16) comprising a plurality of sealed chambers (28) comprising chamber fluid.
